# EUROPEAN PATENT APPLICATION

(11) **EP 2 085 407 A1**
(43) Date of publication of application: **05.08.2009**
(21) Application number: 08002029.0
(22) Date of filing: 04.02.2008
(51) Int. Cl.: C07K 16/28, A61K 39/395, A61P 37/00

(54) **Treatment of idiopathic thrombocytopenic purpura**

(71) Applicant: Sahltech I Göteborg AB, 412 88 Göteborg (SE)
(72) Inventor: Olsson, Bob, 41320 Göteborg (SE); Wadenvik, Hans, 431 69 Mölndal (SE)
(74) Representative: Becker Kurig Straus

(57) **Abstract**

The present application pertains to the treatment of idiopathic thrombocytopenic purpura (ITP) employing pharmaceutical compositions containing a VLA-4 antagonist as active ingredient. The VLA-4 antagonists may be in form of chemical compounds or antibodies specifically blocking VLA-4 activity avoiding side-effects.

## Description

The present invention pertains to the treatment of idiopathic thrombocytopenic purpura (ITP) employing pharmaceutical compositions containing a VLA-4 antagonist as active ingredient. The VLA-4 antagonists may be in form of chemical compounds or antibodies specifically blocking VLA-4 activity avoiding or minimizing side-effects.

Idiopathic thrombocytopenic purpura (ITP) is an autoimmune disease where platelets are destroyed prematurely. Besides the well-known autoantibody-mediated destruction of platelets in the reticuloendothelial systems (Harrington WJ et al., J Lab Clin Med., 38 (1951) 1-10; Dixon RH, Rosse WF, Br J Haematol., 31 (1975) 129-134; McMillan R., N Engl J Med., 304 (1981) 1135-1147; and van Leeuwen EF et al., Blood, 59 (1982) 23-26), several T-cell abnormalities have been identified in ITP. CD4⁺ T-helper cells from ITP patients have been shown to secrete interleukin 2 (IL-2) upon stimulation with autologous platelets (Semple JW, Freedman J., Blood, 78 (1991) 2619-2625; Ware RE, Howard TA., Blood, 82 (1993) 2137-2142). Clonal expansion of CD4⁺ T-helper cells has also been reported (Shimomura T et al., Br J Haematol., 95 (1996) 732-737) and it has been shown that T-cells from ITP patients can proliferate in vitro by GPIIb/IIIa stimulation (Kuwana M et al., J Clin Invest., 102 (1998) 1393-1402; Sukati H et al., Blood; (109) 4528-4538). It has been demonstrated that platelets in ITP can be directly destroyed by CD8⁺ T-cell mediated cytotoxicity (Olsson B et al., Nat Med., 9 (2003) 1123-1124), and that activation-induced cell death (AICD) of T-cells is impaired (Olsson B et al., Thromb Haemost., 93 (2005) 139-144). It has been shown that ITP during active phase is associated with a Th1 cytokine profile (Semple JW et al., Blood, 87 (1996) 4245-4254; Mouzaki A et al., Blood, 100 (2002) 1774-1779), i.e. secretion of interferon gamma (IFN-γ) and tumor necrosis factor alpha (TNF-α), whereas remission is associated with an elevated level of transforming growth factor beta 1 (TGF-β1) (Mouzaki A et al., Blood, 100 (2002) 1774-1779; Andersson PO et al., Ann Hematol., 79 (2000) 507-513), i.e. a Th3 profile.

In patients suffering from ITP, platelets are mainly destroyed in the spleen, liver and bone marrow. They often have purple bruises that appear on the skin or on the mucous membranes. A person who has ITP may also have bleeding resulting in tiny red or purple dots on the skin, the so-called petechiae. People with ITP may have nosebleeds, bleeding from the gums when they have dental work done, or other bleeding that's hard to stop. Women with ITP may have heavy menstrual bleeding. Bleeding in the brain as a result of ITP is very rare, but life threatening.

Two types of ITP, an acute and a chronic form exist. Acute ITP generally lasts less than 6 months. It mainly occurs in children and represents the most common type of ITP. It often occurs after viral infection. Chronic ITP is long-lasting (6 months or longer) and mostly affects adults. Chronic ITP affects women 2 to 3 times more often than men.

Treatment of ITP depends on severity of the bleeding symptoms and the platelet count. Medical treatments comprise e.g. administration of corticosteroids, such as prednisone, acting as immuno suppressant. Drawbacks of the above mentioned treatments reside on the one hand in side effects associated with administration of steroids. It has also been reported that patients may experience relapse after treatment interruption. In certain cases the administration of medicaments is performed along with splenectomy.

An objective of the present invention therefore resides in the treatment of ITP avoiding side-effects. Another objective is seen in the provision of a treatment of ITP avoiding the risk of relapse. Still another objective is the provision of a treatment of ITP avoiding surgical steps.

The above mentioned objectives have been overcome by providing a VLA-4 antagonist for use as an ITP medicament.

During the extensive studies leading to the present invention the present inventors surprisingly found that ITP patients had an accumulation of T-cells in bone marrow, but not in blood. This finding coincides with an increased mRNA and surface expression of VLA-4 and CX3CR1 indicating that changes in T-cell trafficking are involved in the pathogenesis of ITP in that VLA-4 and to a minor extent fractalkine (CX3CR1) may be novel targets for treatment and/or prevention of ITP. Without wishing to be bound by any theory it is presently assumed that due to an increased surface expression of VLA-4 and CX3CR1, T-cells from ITP patients relocate from peripheral blood and accumulate in the bone marrow. In addition, the ITP patients have increased number of activated T-cells but fewer regulatory T-cells in their bone marrow suggesting that preliminary VLA-4 is important for the homing of effector T-cells to the organs involved in the destruction of platelets in ITP, e.g. spleen liver and bone marrow. The same effect may be allocated to a minor degree to CX3CR1. The increased number of T-cells found in bone marrow of ITP patients compared with controls emphasizes the importance of T-cells in the pathogenesis of the disease.

### In the figures

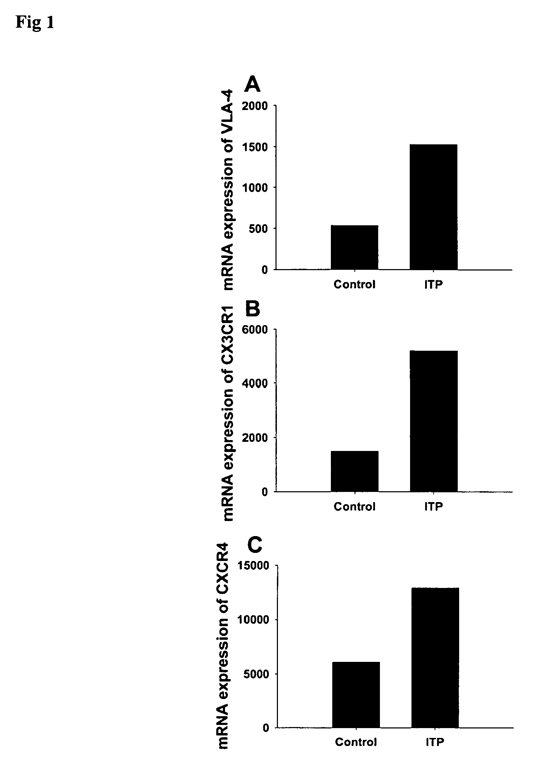

Figure 1 shows that mRNA expression of VLA-4, CX3CR1 and CXCR4 is increased in peripheral blood T-cells of ITP patients. The results are presented as mean DNA microarray expression of (A) VLA-4, (B) CX3CR1 and (C) CXCR4. Data are from 2 separate cDNA synthesis and microarray hybridizations from pooled RNA, isolated from peripheral blood T-cells of 5 ITP patients with active disease (platelet count<50x10⁹ cells/l) and 5 healthy controls.

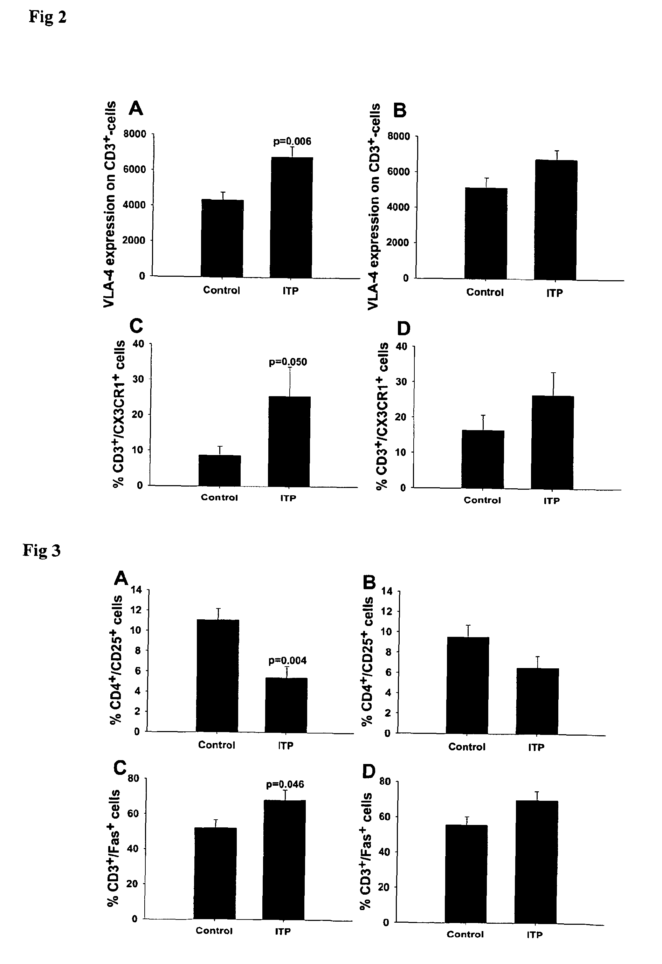

Figure 2 shows that surface expression of VLA-4 and CX3CR1 is increased in bone marrow T-cells of ITP patients. Flow cytometric analysis of VLA-4 and CX3CR1 on CD3⁺ T-cells in peripheral blood (A, C) and bone marrow (B, D) from ITP patients (n=6) and controls (n=8). Data are presented as mean±SEM.

Figure 3 shows reduced number of CD4⁺/CD25^{bright} regulatory T-cells and increased Fas expression in ITP. Flow cytometric analysis of CD25 on CD3⁺/CD4⁺ T-helper cells and Fas on CD3⁺ T-cells in peripheral blood (A, C) and bone marrow (B, D) from ITP patients (n=6) and controls (n=8). The results are given as percentage of T-cells expressing CD25 and Fas. Data are presented as mean±SEM.

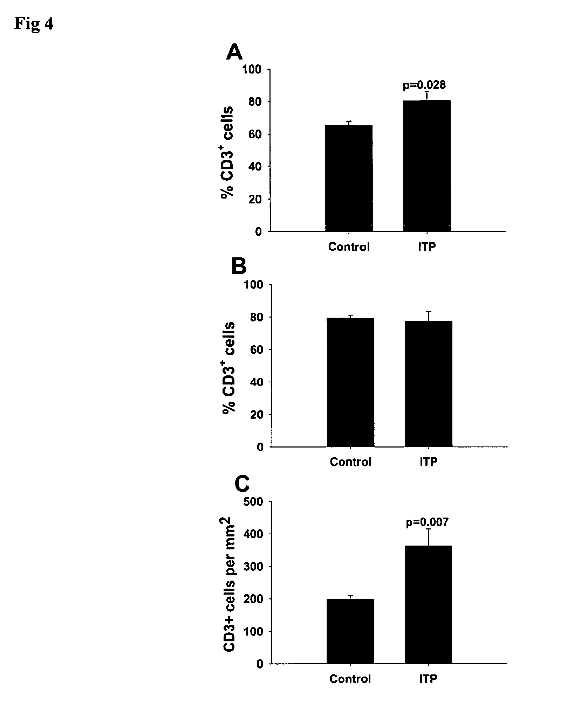

Figure 4 shows an increased number of T-cells in bone marrow of ITP patients. Flow cytometric analysis of CD3⁺ T-cells in blood (A) and bone marrow (B) from ITP patients (n=5) and controls (n=6). The results are given as percentage CD3⁺ cells within the lymphocyte population gated in the forward and side scatter dot plot. Immunohistochemistry (C) and enumeration of CD3⁺ T-cells on bone marrow biopsies obtained from untreated and newly diagnosed ITP patients (n=17) and controls (n=7). These results are given as number of CD3⁺ cells per cross-sectional area (mm²). Data are presented as mean±SEM.

According to a preferred embodiment, the present invention pertains to a VLA-4 antagonist for use as an idiopathic thrombocytopenic purpura (ITP) medicament.

The VLA-4 antagonist is useful for both treatment of ITP and/or prevention of ITP e.g. after a patient has experienced a relapse.

The medicament may be for example in form of a pharmaceutical composition comprising a pharmaceutically acceptable carrier.

The very late antigen-4 (VLA-4; CD49d/CD29) is an integrin expressed on all leukocytes, except platelets and mature neutrophils, including dendritic cells and macrophage-like cells and represents a key mediator of the cell-cell and cell-matrix interactions of these cell types. Ligands for VLA-4 include vascular cell adhesion molecule-1 (VCAM-I), the CS-I domain of fibronectin (FN), and the matrix protein, osteopontin. Neutralizing anti-[alpha]4 antibodies or blocking peptides that inhibit the interaction between VLA-4 and its ligands have been shown to be efficacious both prophylactically and therapeutically in animal models of disease including asthma, multiple sclerosis, inflammatory bowel disease, multiple myeloma, and rheumatoid arthritis.

Suitable VLA-4 antagonists are for example Natalizumab, 2,6-quinolinyl and 2,6-naphthyl derivatives, imidazolone phenylalanine derivatives and other compounds disclosed e.g. in WO 2006/113199, EP 1 870 402, WO 2006/127584, WO 2006/010054 and WO 03/099809, the contents of which are incorporated by way of reference.

Other VLA-4 antagonists are monoclonal or polyclonal antibodies. These antibodies may be commercial, such as mouse anti-VCAM-1 (CD106) monoclonal antibody, clone P8B1 (Millipore Corporation, Cat. No. MAB2511); unconjugated mouse anti-VCAM-1 (CD106) monoclonal antibody, clone P1B8 (Millipore Corporation, Cat. No. MAB2511Z); unconjugated mouse anti-human integrin alpha 4 / VLA-4 / CD49d monoclonal antibody, clone 2B4 (R&D Systems, Cat. No. BBA37); and unconjugated mouse anti-integrin beta 1 monoclonal antibody, unconjugated, clone BV7 (GeneTex, Cat. No. GTX27168).

The preparation of monoclonal or polyclonal antibodies and particularly of antibodies directed to VLA-4 are well known to the skilled person. The preparation of suitable VLA-4 antibodies is for example disclosed e.g. in US 2007/248598 and JP 2006/290897, the contents of which are incorporated by way of reference.

According to another embodiment the VLA-4 antagonist is contained in the composition in an amount of 0.25 % to 15% by weight, preferably in an amount of 0.5 % to 8 % by weight, more preferably in an amount of 1 % to 3 % by weight.

According to another preferred embodiment said composition further comprises a fractalkine (CX3CR1) antagonist.

Fractalkine is the only member of the CXXXC chemokine subfamily exhibiting distinct characteristics in the structure and functions thereof that are not found in other chemokines. Fractalkine is expressed on a cell surface as a membrane-bound protein having a chemokine domain, mucin domain, transmembrane domain and intracytoplasmic domain. The membrane-bound fractalkine by itself can mediate strong adhesion by binding to CX3CR1 even in the presence of a physiological blood flow rate without mediation of selectin or integrin. That is, by a one-stage reaction, the fractalkine-CX3CR1 cell infiltration system mediates a function similar to that of the multistage cell infiltration mechanism via selectin or integrin. Further, secretory fractalkine secreted from membrane-bound fractalkine by shedding binds to CX3CR1 and induces integrin activation and cell migration like the known chemokines.

Preliminary studies surprisingly suggested synergistic effects in case the composition used as an ITP medicament comprises both aVLA-4 and CX3CR1 antagonist. In addition to the up-regulation of VLA-4, also an increased expression of CX3CR1 in peripheral T-cells from ITP patients by DNA microarray analysis was found in the study leading to the present invention. The increased T-cell expression of CX3CR1 in ITP was confirmed in bone marrow but not in blood by flow cytometry. Fractalkine levels were elevated in bone marrow plasma, compared with blood, in ITP patients and control groups. This suggests that, in ITP, the increased expression of CX3CR1 alone may be a sufficient chemokine signal for trafficing of T-cells from peripheral blood into the bone marrow. CX3CR1 is expressed on NK-cells and CD8+ T-cells, and most of them are positive for granzyme B. CX3CR1 appears to be the main receptor for homing of CD8⁺ cells in AIDS. The interaction between fractalkine and CX3CR1 on effector lymphocytes in endothelium is enough for adhesion and tissue recruitment. Theses findings indicate that CX3CR1 may contribute to the recruitment of T-cells, and especially CD8⁺ cytotoxic cells, to the effector organs for platelet destruction in ITP. An increased number of CD3⁺/CD8⁺/CX3CR1⁺ cells were also observed in bone marrow of ITP patients compared with controls. Together these data indicate that the CD8⁺ T-cells residing in the bone marrow participate in the destruction of platelets in ITP by T-cell mediated cytotoxicity.

Compounds blocking or neutralizing CX3CR1 as well as the preparation of monoclonal or polyclonal antibodies and particularly of antibodies directed to CX3CR1 are well known to the skilled person. The preparation of suitable CX3CR1antibodies is for example disclosed e.g. in EP1806145, the contents of which are incorporated by way of reference.

According to an embodiment the CX3CR1 antagonist is contained in the composition in an amount of 0.25 % to 15% by weight, preferably in an amount of 0.5 % to 8 % by weight, more preferably in an amount of 1 % to 3 % by weight.

According to another embodiment medicament is in form of pharmaceutical composition comprising a pharmaceutically acceptable carrier or medicament, which may be in form of an ointment, liquid, gel, gelcap, capsule, powder, solid tablet (coated or non-coated), dried oral supplement, wet oral supplement, or the like. The carrier is preferably in the form of a tablet or capsule and most preferably in the form of a hard gelatin capsule. Suitable excipient and/or carriers include maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and the like (including mixtures thereof).

Preferred carriers include calcium carbonate, magnesium stearate, maltodextrin, and mixtures thereof. The various ingredients and the excipient and/or carrier are mixed and formed into the desired form using conventional techniques. The tablet or capsule of the present invention may be coated with an enteric coating that dissolves at a pH of about 6.0 to 8.0, preferably at a pH of about 6.0 to 7.0. A suitable enteric coating that dissolves in the small intestine but not in the stomach is cellulose acetate phthalate. Further details on techniques for formulation for and administration may be found in the latest edition of Remington's Pharmaceutical Sciences (Maack Publishing Co., Easton, Pa.).

Other suitable pharmaceutically acceptable carriers for use with the present invention include, but are not limited to, water, mineral oil, ethylene glycol, propylene glycol, lanolin, glyceryl stearate, sorbitan stearate, isopropyl myristate, isopropyl palmitat, acetone, glycerol, phosphatidylcholine, sodium cholate, or ethanol.

The composition according to the invention can also comprise at least one co- emulsifier, which includes, but is not restricted to, oxyethylenated sorbitan monostearate, fatty alcohols such as stearyl alcohol or cetyl alcohol, or esters of fatty acids and polyols such as glyceryl stearate.

Preferably, the pharmaceutical compositions of the present invention are stabilized. In general, stabilization methodologies and techniques that may be used in accordance with the present invention include any and all methods for the stabilization of chemical or biological material known to the art, including e.g. the addition of chemical agents, temperature modulation based methodologies; radiation based methodologies or combinations thereof. Chemical agents that may be used in accordance with the present invention include inter alia preservative agents; acids; bases; salts; anti-oxidants; viscosity modifying agents; emulsifiers; gelling agents; and mixtures thereof.

A wide variety of emulsifiers can be used to provide a beneficial effect in the present compositions, and the particular levels of use in connection with each type or class of emulsifiers can be readily determined. In general, a selected emulsifier or emulsifier system should be capable of maintaining different components of the nutritional composition in a stable dispersion, when the base is dispersed in an aqueous medium. In most cases when the base is dispersed in water, the emulsifier should be capable of forming stable oil-in-water emulsions. In particular, the emulsifier is preferably selected from the group consisting of: stearyl-n-lactylic acids, where n ranges from about 1 to 5, and the sodium, potassium and calcium salts thereof, succinylated mono- and diglycerides of edible C12-C24 fatty acids and the sodium and potassium salts thereof, diacetyl tartaric acid esters of mono- and diglycerides of C12-C24 edible fatty acids, and the sodium and potassium salts thereof, polyglycerol esters of C12-C24 edible fatty acids, ranging from 3 to 10 glycerol units and one to ten fatty acids per molecule, polyoxyethylene (20) sorbitan mono-, di- and triesters of C12-C24 edible fatty acids, ethoxylated mono- and diglycerides of edible C12-C24 fatty acids, stearyl monoglyceridyl citrate, and the sodium and potassium salts thereof, citric acid esters of mono- and diglycerides of edible C12-C24 fatty acids, and the sodium and potassium salts thereof, propylene glycol mono- and diesters of edible C12-C24 fatty acids, glycerol mono-and diesters of edible C12-C24 fatty acids, lactylated propylene glycol and glycerol mono-and diesters of edible C12-C24 fatty acids, acetylated propylene glycol and glycerol mono-and diesters of edible C12-C24 fatty acids, sorbitan monostearate, lecithin, sucrose esters of edible C12-C24 fatty acids, phosphated mono- and diglycerides of edible C12-C24 fatty acids, and mixtures thereof. Most preferably, the emulsifier is anionic and is selected from the group consisting of sodium stearyl-2-lactylate, succinylated mono- and diglycerides of edible C12-C24 fatty acids, diacetyl tartaric acid esters of mono- and diglycerides of C12-C24 fatty acid esters in their acid or salt form, and mixtures thereof.

Other compounds suitable for use in combination with a VL4-4 antagonist in the preparation of a respective carrier include one or more of vitamin actives, including but not restricted to vitamin A and derivatives, including retinoic acid, retinyl aldehyde, retin A, retinyl palmitat, adapalene, and beta-carotene; vitamin B (panthenol, provitamin B5, panthenic acid, vitamin B complex factor); vitamin C (ascorbic acid and salts thereof) and derivatives such as ascorbyl palmitat; vitamin D including calcipotriene (a vitamin D3 analog) vitamin E including its individual constituents alpha-, beta-, gamma-, delta-tocopherol and cotrienols and mixtures thereof and vitamin E derivatives including vitamin E palmitat, vitamin E linolate and vitamin E acetate; vitamin K and derivatives; vitamin Q (ubiquinone) and mixtures thereof.

Preferably, the compositions of the present invention are stabilized. In general, stabilization methodologies and techniques that may be used in accordance with the present invention include any and all methods for the stabilization of chemical or biological material known to the art, including e.g. the addition of chemical agents, temperature modulation based methodologies; radiation based methodologies or combinations thereof. Chemical agents that may be used in accordance with the present invention include inter alia preservative agents; acids; bases; salts; anti-oxidants; viscosity modifying agents; emulsifiers; gelling agents; and mixtures thereof.

Compositions of the invention may also include viscosity modifiers, preferably in amounts from about 0.01 to about 10% by weight of the composition. Viscosity modifiers such as cetyl alcohol, glycerol, polyethylene glycol (PEG), PEG-stearate, or Keltrol may also be used to enhance the stability of the formulation. Thickeners which may enhance the stability include gelling agents such as cellulose and derivatives, Carbopol and derivatives, carob, carregeenans and derivatives, xanthane gum, sclerane gum, long chain alkanolamides, bentone and derivatives, Kaolin USP, Veegum Ultra, Green Clay, Bentonite NFBC, magnesium aluminum silicate (Veegum@), guar gums (such as JaguarHP-120 @), xanthan gum, sodium caroxymethyl cellulose, hydroxyalkyl and alkyl celluloses, cross-linked acrylic acid polymers, and mixtures thereof. As known to the skilled person, the precise amount of thickeners can vary depending upon the consistency and thickness of the composition which is desired.

The compositions are administered in an amount to be effective for the intended application and the subject to be treated. To this end, the dosage of the composition and other constituents may vary depending on age, weight, and condition of the subject. In general, the active agent is preferably administered at a concentration that will afford effective results without causing any harmful or deleterious side effects, and may be administered either as a single unit dose, or if desired in convenient subunits administered at suitable times throughout the day.

The composition of the present invention may be administered multiple times a day, for example, from two to five times adding up for the necessary amount for one day, once a day, or continuously for a necessary term.

The following examples illustrate the invention without limiting it thereto.

### Examples

### Materials and methods

### Subjects

In this study a total of 26 ITP patients and 20 healthy controls were used. All patients fulfilled the diagnostic criteria for chronic ITP as evidenced by isolated thrombocytopenia lasting more than 6 months, presence of normal or increased numbers of megakaryocytes in bone marrow biopsies, normal spleen size and no other cause of thrombocytopenia. Patient characteristics are shown in Table 1. All studies were approved by the local ethics committee at Göteborg University and all participants gave informed consent.

**Table 1 Characteristics of ITP patients**

| ID | Age | Sex | Plc | Treatment | Included in: |
|---|---|---|---|---|---|
| 1 | 32 | F | 58 | None | FC, ELISA |
| 2 | 33 | M | 47 | Prednisone 20 mg qd, IVIG | FC, ELISA |
| 3 | 36 | F | 70 | None | FC, ELISA |
| 4 | 53 | M | 53 | None | FC, ELISA |
| 5 | 45 | M | 11 | None | FC, ELISA |
| 6 | 44 | M | 35 | None | FC, ELISA, MA |
| 7 | 59 | M | 11 | Prednisone 5 mg qd | MA |
| 8 | 53 | F | 45 | Prednisone 5 mg qd | MA, IHC |
| 9 | 28 | F | 50 | None | MA |
| 10 | 44 | F | 33 | Prednisone 10 mg qd | MA |
| 11 | 19 | F | 5 | None | IHC |
| 12 | 76 | F | 34 | None | IHC |
| 13 | 64 | F | 28 | None | IHC |
| 14 | 19 | F | 0 | None | IHC |
| 15 | 55 | F | 1 | None | IHC |
| 16 | 61 | M | 18 | None | IHC |
| 17 | 71 | F | 1 | None | IHC |
| 18 | 52 | M | 4 | None | IHC |
| 19 | 67 | M | 8 | None | IHC |
| 20 | 44 | F | 1 | None | IHC |
| 21 | 17 | F | 8 | None | IHC |
| 22 | 73 | F | 50 | None | IHC |
| 23 | 31 | F | 79 | None | IHC |
| 24 | 60 | M | 44 | None | IHC |
| 25 | 22 | F | 41 | None | IHC |
| 26 | 35 | F | 47 | None | IHC |

| | | | | | |
|---|---|---|---|---|---|
| Abbreviations: FC: flow cytometry; MA: microarray analysis; IHC: immunohistochemistry; IVIG: intravenous immunoglobulins | | | | | |

### Preparation of peripheral blood mononuclear cells (PBMC) and T-lymphocytes for microarray analysis

PBMC were prepared from heparinised blood from 5 ITP patients with active disease (platelet count<50x10⁹ cells/l) and 5 healthy controls by density gradient centrifugation using Ficoll. Monocytes were removed by anti-CD14⁺ magnetic microbeads and T-cells were isolated from the remaining cells using anti-CD3⁺ magnetic microbeads, according to the manufacturer's recommendations (MACS; Miltenyi Biotec, Surrey, UK).

### Preparation of RNA and hybridization to DNA microarrays

RNA was isolated from the CD3⁺ T-cell preparations using the Chomczynski method,¹⁶ followed by RNeasy clean up (Qiagen, Hilden, Germany). The RNA concentration was measured spectrophotometrically with an A260/A280 ratio of 1.8-2.0, and the quality was verified by agarose gel electrophoresis. The RNA from the 5 individuals in each group, i.e. ITP patients and controls, was pooled in equal amounts. Each pool was analyzed in duplicates as described.¹⁰ Briefly, RNA was transcribed into cDNA (Life Technologies, Rockville, MD) and biotin-labeled cRNA (Enzo, Farmingdale, NY) was resynthesized. Hybridization to DNA microarrays (Human Genome U95A array version 2; Affymetrix, Santa Clara, CA) and detection of hybridized target cRNA was performed according to the Affymetrix Gene Chip Expression Analysis manual. Quality of the cDNA-synthesis and in vitro transcription was assessed by hybridization to Test2-arrays (Affymetrix).

### Data analysis

Scanned output files were visually inspected for hybridization artifacts and then analyzed with MicroarraySuite 5.0 software (Affymetrix). To identify regulated genes the duplicate arrays were scaled to an average intensity of 500 and compared in a crosswise fashion¹⁷ using a change call parameter in the MicroarraySuite 5.0 software. These experiments comply with Minimum Information About a Microarray Experiment (MIAME)¹⁸ and all files have been deposited in the gene expression omnibus database (GSE574).

### Flow cytometry

PBMC were isolated from EDTA anticoagulated blood and bone marrow specimens by Ficoll-separation. T-cell expression of CX3CR1, VLA-4, CXCR4, Fas and CD25 was analyzed in a 5 color combination using anti-CD3-Peridine-Chlorophyll-Cy5.5 (PerCP), anti-CD4-fluorescein isothiocyanate (FITC), anti-CD8-allophycocyanin-Cy7 (APC-Cy7), CD16-PE-Cy7 and one of the following antibodies: anti-CX3CR1-phycoerythrin (PE), anti-CD49d-PE (VLA-4), anti-CD 184-PE (CXCR4), anti-CD95-PE (Fas) or anti-CD25-PE. The number of T-cells, B-cells and monocyte/macrophages in blood and bone marrow was determined by a 5 color combination using anti-CD3-PerCP, anti-CD 19-FITC, anti-CD45-APC-Cy7, CD 16-PE-Cy7 and anti-CD 14-APC. Naïve T-cells were identified using antibodies against CD 11 a-APC and CD45Ra-FITC. All monoclonal antibodies (MoAbs) were from Becton Dickinson Bioscience (San Diego, CA) except the antibody for CX3CR1 which was from Nordic Biosite (Täby, Sweden). The samples were analyzed with a FACSCanto (Becton Dickinson, Mountain View, CA) and data were analyzed using the FACSDiva software (Becton Dickinson). First a gate for lymphocytes was set using forward and side scatter, followed by a gate for CD3 and CD 16 identifying T-cells as CD3⁺/CD16⁻. T-helper cells were identified as CD3⁺/CD4⁺ and cytotoxic T-cells as CD3⁺/CD8⁺.

### Analysis of fractalkine (CX3CL1) levels in blood an bone marrow plasma

The levels of fractalkine were analyzed in EDTA-anticoagulated blood and bone marrow plasma obtained from 6 ITP patients and 7 healthy individuals using a commercial available assay (R&D systems Europe Ltd., Abingdon, UK).

### Immunohistochemistry of bone marrow biopsies

Bone marrow biopsies from the iliac crest were available in 17 ITP patients and 7 healthy subjects which served as controls. Five micron thick sections from the paraffin embedded biopsies were stained with haematoxylin-eosin and immunostained with antibodies against CD3, CD4, CD8 and CD20 using the Dako Envision System in a Techmate Horizon Autostainer (Glostrup, Denmark). The number of positively stained cell profiles was enumerated in a Nikon Optiphot-2 microscope at a magnification of 720 x. At least 15 fields of vision, corresponding to a specimen area of 1.2 mm² were examined.

### Statistics

Unless otherwise stated, mean values ± SEM are reported. Differences between groups were evaluated using Student's T-test. A P-value ≤ 0.05 was considered significant.

### Results

### DNA microarray analysis

It could be shown that T-cells both directly and indirectly are involved in the pathogenesis of ITP and scintigraphy of ITP patients receiving radiolabeled platelets have demonstrated that the destruction of platelets occur mainly in the spleen, liver and bone marrow. This suggests that translocation of T-cells from peripheral blood to effector organs may be important in ITP. This led us to investigate the expression of molecules involved in T-cell homing in ITP patients and controls. By DNA microarray analysis of peripheral blood T-cells we found that the mRNA expression of the integrin VLA-4 and the chemokine receptors CX3CR1 and CXCR4 was increased in ITP patients compared with controls (Fig 1).

### Flow cytometric analysis of homing receptors, activation markers and regulatory T-cells in blood and bone marrow of ITP patients and controls

Knowing that bone marrow is one of the effector organs for platelet destruction in ITP, the homing receptors detected in our microarray screen, activation markers and the number of regulatory T-cells (Tregs, CD4⁺/CD25⁺) in blood and bone marrow of ITP patients and controls were analyzed by flow cytometry.

In peripheral blood, there was no difference in the surface expression of CX3CR1 or CXCR4 on T-cells. However, the mean fluorescence intensity of VLA-4 on peripheral blood T-cells was increased in ITP compared with controls (Fig 2). Furthermore, in bone marrow, we found an increased percentage of CD3⁺CX3CR1⁺ cells and increased mean fluorescence intensity of VLA-4 on T-cells from ITP patients compared with controls (Fig 2). The surface expression of CXCR4 on T-cells was not different between ITP patients and controls. Based on that bone marrow is a major reservoir of CD8⁺ central memory T-cells¹⁹ and our previous findings of increased T-cell mediated cytotoxicity towards platelets in ITP,¹⁰ we analyzed VLA-4 and CX3CR1 on CD8⁺ cytotoxic T-cells in bone marrow. Both the mean fluorescence intensity of VLA-4 on CD3⁺/CD8⁺ cells and the percentage of CD3⁺/CD8⁺/CX3CR1⁺ cells were increased in ITP patients compared to controls (VLA-4: controls 4500±342, ITP 6762±732, P=0.03; CX3CR1: controls 14.65±3.84 %, ITP 35.58±9.29 %, P=0.04).

Based on the finding that the mRNA expression of Fas is increased in peripheral T-cells in blood of ITP patients and that T-cells rapidly induce the expression of Fas when activated, Fas expression as a marker of T-cell activation was investigated. It has been found that Fas was over expressed on the surface of T-cells from bone marrow, but not blood, of ITP patients compared with controls (Fig 3).

In the present study a reduced number of Tregs (CD4⁺/CD25^{bright}) were found in bone marrow, but not in blood, of ITP patients compared with controls (Fig 3).

### Analysis of fractalkine levels in ITP patients and controls

Fractalkine is the endogenous ligand for CX3CR1. Blood and bone marrow plasma levels of fractalkine in ITP patients and controls were analyzed. There was no significant difference in blood or bone marrow plasma levels of fractalkine between ITP patients and controls (blood plasma, 10.4±3.6 vs 5.2±1.4 ng/l; bone marrow plasma, 18.4±8.0 vs 8.9±1.3 ng/l, respectively). However, in both groups the levels of fractalkine were higher in bone marrow plasma compared with blood plasma (P=0.037).

### Analysis of T-cells, B-cells and monocytes/macrophages in blood and bone marrow of ITP patients and controls

The increased surface expression of VLA-4 and CX3CR1 in bone marrow T-cells suggested a recruitment of T-cells into the bone marrow. To investigate this, the number of T-cells, B-cells and monocytes/macrophages was analyzed by flow cytometry in 5 ITP patients and 6 controls. An increased number of T-cells (Fig 4), but unchanged number of B-cells (ITP 5.2±1.4%, control 12.7±3.1%) and CD14⁺ monocytes/macrophages (ITP 3.3±0.94%, control 2.6±0.67%) in the bone marrow of ITP patients was found compared with controls. In blood however, there was no difference in the number of these cell subsets between ITP patients and controls (Fig 4; B-cells 5.1±1.6% vs 5.5±1.0%; monocytes/macrophages 3.5±1.2% vs 4.0±1.2%, ITP and controls respectively). To verify the finding of increased number of T-cells in the bone marrow of ITP patients immunohistochemical staining on bone marrow biopsies obtained from 17 newly diagnosed and untreated ITP patients and 7 healthy controls was performed. Once more, an increased number of T-cells (Fig 4) and unchanged number of B-cells (ITP 234±29, Control 222±30) in the bone marrow sections from ITP patients was found compared with controls. However, the number of T-helper cells (expressing CD4) or cytotoxic T-cells (expressing CD8) was not significantly different between ITP and control (CD4⁺: control 181±30, ITP 218±27 cells/mm²; CD8⁺: control 157±21, ITP 218±36 cells/mm²).

To investigate whether the increase in CD3⁺ cells in the bone marrow of ITP patients was due to an increased production of native T-cells the surface expression of CD11a and CD45Ra in ITP patients and controls as previously described (Heydtmann M et al., J Immunol., 177 (2006) 729-738) was analyzed.

No difference in the number of native T-cells between ITP patients and controls was found (data not shown).

## Claims

1. A VLA-4 antagonist for use as an idiopathic thrombocytopenic purpura (ITP) medicament.

2. The use according to claim 1, wherein said VLA-4 antagonist is an antibody selected from the group consisting of unconjugated Mouse Anti-VCAM-1 (CD106) Monoclonal Antibody, Clone P8B1; unconjugated Mouse Anti-VCAM-1 (CD106) Monoclonal Antibody, Clone P1B8; unconjugated Mouse Anti-Human Integrin alpha 4 / VLA-4 / CD49d Monoclonal Antibody, Clone 2B4; and unconjugated Mouse Anti-Integrin beta 1 Monoclonal Antibody, Unconjugated, Clone BV7.

3. The use according to any of the proceeding claims, wherein said VLA-4 antagonist is contained in an amount of 0.25 % to 15% by weight.

4. The use according to any of the proceeding claims, further comprising a CX3CR1 antagonist.

5. The use according to claim 4, wherein said CX3CR1 antagonist is contained in an amount of 0.25 % to 15% by weight.

6. The use according to any of the proceeding claims, wherein the medicament is in form of pharmaceutical composition comprising a pharmaceutically acceptable carrier, which may be in form of a liquid, gel, gelcap, capsule, powder, solid tablet, dried oral supplement or wet oral supplement.
